(19) **Europäisches Patentamt / European Patent Office / Office européen des brevets**

(11) **EP 2 644 620 B1**

(12) **EUROPEAN PATENT SPECIFICATION**

(45) Date of publication and mention
of the grant of the patent:
**28.06.2017 Bulletin 2017/26**

(51) Int Cl.:
*C07K 14/78* (2006.01)   *A61L 15/16* (2006.01)
*A61L 27/00* (2006.01)   *A61L 31/00* (2006.01)

(21) Application number: **11843692.2**

(22) Date of filing: **28.11.2011**

(86) International application number:
**PCT/JP2011/077425**

(87) International publication number:
**WO 2012/070680 (31.05.2012 Gazette 2012/22)**

(54) **NON-FIBROGENESIS COLLAGEN MATERIAL AND A MANUFACTURING METHOD THEREOF**

NICHT-FIBROGENESE-KOLLAGEN UND METHOD ZU SEINER HERSTELLUNG

COLLAGÈNE NON-FIBROGÈNE ET MÉTHODE POUR SA PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.11.2010 JP 2010264376**

(43) Date of publication of application:
**02.10.2013 Bulletin 2013/40**

(73) Proprietor: **Tokyo Institute of Technology**
**Tokyo 152-8550 (JP)**

(72) Inventors:
• **TANAKA Junzo**
**Tokyo 152-8550 (JP)**
• **IKOMA Toshiyuki**
**Tokyo 152-8550 (JP)**
• **YOSHIOKA Tomohiko**
**Tokyo 152-8550 (JP)**

(74) Representative: **Gille Hrabal**
**Brucknerstrasse 20**
**40593 Düsseldorf (DE)**

(56) References cited:
WO-A1-01/92322   JP-A- 2006 257 013
JP-A- 2006 257 014   US-A- 3 368 911

• CHEN S ET AL: "Microstructures and rheological properties of tilapia fish-scale collagen hydrogels with aligned fibrils fabricated under magnetic fields", ACTA BIOMATERIALIA, ELSEVIER, AMSTERDAM, NL, vol. 7, no. 2, 1 February 2011 (2011-02-01), pages 644-652, XP027577075, ISSN: 1742-7061 [retrieved on 2010-12-27]
• I. KARUBE ET AL: "Electrochemical aggregation of tropocollagen", BIOCHEMICAL AND BIOPHYSICAL RESEARCH COMMUNICATIONS, vol. 48, no. 2, 1 July 1972 (1972-07-01), pages 320-325, XP055107098, ISSN: 0006-291X, DOI: 10.1016/S0006-291X(72)80053-6
• P. F. DAVISON: "THE SEROLOGIC SPECIFICITY OF TROPOCOLLAGEN TELOPEPTIDES", JOURNAL OF EXPERIMENTAL MEDICINE, vol. 126, no. 2, 1 August 1967 (1967-08-01), pages 331-346, XP055107066, ISSN: 0022-1007, DOI: 10.1084/jem.126.2.331
• TOSHIYUKI IKOMA ET AL: "Physical properties of type I collagen extracted from fish scales of Pagrus major and Oreochromis niloticas", INTERNATIONAL JOURNAL OF BIOLOGICAL MACROMOLECULES, ELSEVIER BV, NL, vol. 32, no. 3-5, 1 September 2003 (2003-09-01), pages 199-204, XP002626600, ISSN: 0141-8130, DOI: 10.1016/S0141-8130(03)00054-0 [retrieved on 2003-07-19]

- SKIERKA ET AL: "The influence of different acids and pepsin on the extractability of collagen from the skin of Baltic cod (Gadus morhua)", FOOD CHEMISTRY, ELSEVIER LTD, NL, vol. 105, no. 3, 1 January 2007 (2007-01-01), pages 1302-1306, XP022158247, ISSN: 0308-8146, DOI: 10.1016/J.FOODCHEM.2007.04.030
- JUNJIE ZHANG ET AL: "ISOLATION AND CHARACTERIZATION OF COLLAGENS FROM SCALE OF SILVER CARP (HYPOPHTHALMICHTHYS MOLITRIX)", JOURNAL OF FOOD BIOCHEMISTRY, vol. 34, no. 6, 18 December 2010 (2010-12-18), pages 1343-1354, XP055107104, ISSN: 0145-8884, DOI: 10.1111/j.1745-4514.2010.00439.x
- NOBUHIRO OGAWA ET AL.: 'Uroko I Gata Collagen o Mochiita Saibo Baiyoyo Maku Zairyo no Soshutsu' DAI 32 KAI THE ANNUAL MEETING OF THE JAPANESE SOCIETY FOR BIOMATERIALS YOKOSHU 29 November 2010, page 380, XP008168366
- REINA MATSUMOTO ET AL.: 'Sakana Uroko Collagen-coated Dishes o Mochiita Hito Kan'yokei Kansaibo no Bunka Katei no Kenkyu' DAI 32 KAI THE ANNUAL MEETING OF THE JAPANESE SOCIETY FOR BIOMATERIALS YOKOSHU 29 November 2010, page 129, XP008168365
- TOSHIMASA UEMURA ET AL.: 'Sakana Uroko Collagen o Mochiita Saibo Baiyo' REGENERATIVE MEDICINE vol. 10, no. 2011, 01 February 2011, page 262, XP008168360
- NORIFUMI AZUMA ET AL.: 'Gyorin Yurai Type-I Collagen no Netsurikigakuteki Anteisei no Seigyo' JAPANESE SOCIETY FOR BIOMATERIALS 2004, page 242, XP008168363
- ATSUSHI MATSUDA ET AL.: 'Seibutsu Kino Material: Umi Kara no Okurimono o Ikasu' MATERIALS INTEGRATION vol. 20, no. 5, 2007, pages 11 - 16, XP008168359
- TOSHIYUKI IKOMA ET AL.: 'Collagen no Nano-technology' BIO INDUSTRY vol. 28, no. 11, 12 October 2011, pages 16 - 21, XP008168361

**Description**

TECHNICAL FIELD

The present invention relates to a non-fibrogenesis collagen material and a manufacturing method thereof, particularly, a non-fibrogenesis collagen transparent membrane and a non-fibrogenesis collagen porous material; and a manufacturing method thereof. The non-fibrogenesis collagen material, such as the non-fibrogenesis collagen transparent membrane or the non-fibrogenesis collagen porous material, of the present invention may be used as a cell culture substrate, a scaffold material for regenerative medicine, an implantation material, and a carrier for drug delivery.

BACKGROUND ART

Collagen is an important protein that contributes 30% of all proteins in a living body and functions as support for bone and cell adherence. For example, collagen is a main constituent element in tissues such as bone, cartilage, ligament, tendon, corneal stroma, skin, liver, muscle, and the like of the human body. Thus, collagen material (biomaterial) is useful for a cell culture substrate, a scaffold material for regenerative medicine (material for tissue engineering of cartilage, bone, ligament, corneal stroma, skin, or liver, for example), an implantation material (wound dressing material, bone grafting material, hemostatic material, or anti-adhesive material, for example) or a carrier for drug delivery. In particular, the collagen material is absolutely imperative for huge tissue regeneration via regenerative medicine. However, a mechanical characteristic of collagen material is not satisfactory and thus, there is a limit to clinical use thereof.

Hitherto, as for collagen material, the materials containing collagen fibers obtained by a fibrogenesis from soluble collagen in vitro are reported. For example, Non-Patent Reference 1 and Non-Patent Reference 2 disclose that a collagen-thin membrane containing collagen fiber is obtained via a bovine-derived collagen. The collagen-thin membrane has a certain level of strength due to vitrification (drying for at least two weeks). However, the strength of the collagen-thin membrane is not still sufficient. Therefore, the collagen-thin membrane, wherein the outer edge is reinforced by a nylon frame, is only commercially available as a cell culture substrate (Non-Patent Reference 1 and Non-Patent Reference 2). Further, Patent Reference 1 discloses a collagen membrane obtained by fibrogenesis from fishskin-derived collagen, freeze-drying an obtained collagen gel containing the collagen fibers, and then cross-linking the freeze-dried gel with thermal cross-linking or chemical cross-linking (using a solution of carbodiimide, glutaraldehyde, succinimide or the like). Further, Patent Reference 2 discloses a stretchable collagen material in a solution obtained by simultaneous fibrogenesis from fishskin-derived collagen and chemical cross-linking with a crosslinking agent.

The above materials contain collagen fibers, and further mechanical strengths of these materials were increased by the vitrification or cross-linking thereof. The collagen-thin membrane disclosed in Non-Patent Reference 1, wherein collagen fibers with high density are tangled together, is vitrified.

However, the strengths of the collagen materials are not sufficient as scaffold materials for regenerative medicine or implantation materials. For example, the collagen-thin membrane disclosed in Non-Patent References 1 and 2, wherein the outer edge is reinforced by a nylon frame, is only commercially available as a cell culture substrate.

Some further prior arts for collagen materials can be seen in Patent Reference 3, Non-Patent References 3 and 4.

CITATION LIST

PATENT LITERATURE

[Patent literature 1] Japanese Translation Publication (Kohyo) No. 2003-534858
[Patent literature 2] Japanese Unexamined Patent Publication (Kokai) No. 2005-334625
[Patent literature 3] International Patent Publication WO 01/92322 A1

NON-PATENT LITERATURE

[NON-PATENT LITERATURE 1] YAKUGAKU ZASSHI (Japan) 2010, vol.130, p.565-574
[NON-PATENT LITERATURE 2] Journal of BIOTECHNOLOGY (Germany) 2007, vol.131, p.76-83
[NON-PATENT LITERATURE 3] Acta Biomaterialia, vol. 7, 2011, p.644-652, Chen et al.
[NON-PATENT LITERATURE 4] Biochemical and Biophysical Research Communications, vol. 2, 1972, p320-325

SUMMARY OF INVENTION

TECHNICAL PROBLEM

Hitherto, the strength of the collagen material is increased by collagen fibrogenesis as disclosed in Non-Patent References 1 and 2. However, the material has poor transparency, although the collagen material has the increased strength due to the collagen fibrogenesis. For example, the collagen membrane is expected to be used as an implantation material for corneas. However, the implantation material for a cornea needs above certain transparency and strength levels.

Therefore, the object of the present invention is to provide a non-fibrogenesis collagen material having above certain transparency and strength levels.

Specifically, the object of the present invention is to provide a non-fibrogenesis collagen material which has a sufficient strength and can be used as a cell culture substrate, a scaffold material for regenerative medicine (for example, material for tissue engineering of cartilage, bone, ligament, corneal stroma, skin, or liver), an implantation material (for example, wound dressing material, bone grafting material, hemostatic material, anti-adhesive material) or a carrier for drug delivery. For example, one of the objects of the present invention is to provide a non-fibrogenesis collagen transparent membrane which can be used as the implantation material for the cornea. Further, the object of the present invention is to provide a collagen membrane with high transparency. When a collagen membrane with high transparency is used as the cell culture substance in a cell culture, cells can be observed through the collagen membrane with usual microscopy. Furthermore, the object of the present invention is to provide a non-fibrogenesis collagen porous material wherein cells can be seeded evenly. Cartilage tissue with high strength and liver tissue with an excellent coefficient of elasticity and a capacity to produce albumin, for example, can be regenerated using the non-fibrogenesis collagen porous material.

SOLUTION TO PROBLEM

Hitherto, the collagen materials were prepared by forming collagen fibers from swine or bovine collagen. Specifically, the collagen materials were prepared in accordance with the following procedure. The swine or bovine collagen was dissolved into an acid solution such as an acetic acid solution or a hydrochloric acid solution. Then, the collagen acid solution was adjusted to an appropriate salt concentration and a neutral pH, and a collagen fibril was formed. Therefore, the resulting material contains salt, and a purity of collagen contained in the resulting material is low. Therefore, the material does not have sufficient strength as a biomaterial. Further, the collagen fibers do not have an orientation, and thus, it is difficult to prepare a collagen fiber membrane with high transparency.

The present invention provides a non-fibrogenesis collagen transparent membrane according to claim 1, as well as some advantageous embodiments as described in dependent claim 2 to 5.

Furthermore, our invention also provides a method for preparing a non-fibrogenesis collagen material according to claim 6, as well as some advantageous embodiments as described in dependent claims 7 to 10.

The present inventors have conducted intensive studies into collagen membranes with high transparency and sufficient strength as a cell culture substrate, a scaffold material for regenerative medicine, or an implantation material. As a result, the present inventors found that a collagen membrane having a high strength and a high transparency can be obtained through the formation of the membrane from a fish-derived collagen, particularly a fish scale-derived collagen, without collagen fibril formation. Further, in order to prepare a highly-transparent and highly-pure membrane, it is important that a collagen gel does not contain a salt and the collagen transparent membrane has a structure wherein non-fibrogenesis collagen molecules are closely-tangled. Furthermore, the present inventors found that the transparent, non-fibrogenesis collagen membrane having a high density and high strength can be prepared by using a collagen acid solution containing carbon dioxide and fish-derived collagen, without collagen fibrogenesis. The transparent, non-fibrogenesis collagen membrane of the present invention has closely-tangled collagen molecules, and exhibits an excellent tensile strength of 30MPa or more. Further, the present inventors found that the strength of the transparent, non-fibrogenesis collagen membrane can be increased by a cross-linking method using glutaraldehyde evaporation, without swelling. Furthermore, the non-fibrogenesis collagen porous material without collagen fibrogenesis obtained by using the fish-derived collagen, has a high porosity and excellent strength.

The term "non-fibrogenesis" as used herein means that collagen molecules do not form collagen fine fibrils. It does not mean that the nano-fiber, wherein each five molecules of type I collagen shifted in the direction of the long axis at 1/4 length are self-assembled, is not formed.

ADVANTAGEOUS EFFECTS OF INVENTION

The non-fibrogenesis collagen material such as the transparent, non-fibrogenesis collagen membrane or non-fibrogenesis collagen porous material of the present invention is useful as a cell culture substrate, a scaffold material for regenerative medicine, an implantation material, or a carrier for drug delivery. Therefore, said invention can solve a problem related to a handling in medical practice caused by a lack of mechanical strength thereof.

For example, the transparent, non-fibrogenesis collagen membrane of the present invention is a high-density, transparent, and non-fibrogenesis collagen membrane which has not existed until now. The transparent and non-fibrogenesis collagen membrane has very high mechanical strength, despite an absence of collagen fibril formation. Further, a tensile strength of the transparent and non-fibrogenesis collagen membrane is dramatically increased by cross-linking. For example, the transparent and non-fibrogenesis collagen membrane having 50MPa or more of tensile strength can be obtained by chemical cross-linking using glutaraldehyde evaporation. Furthermore, a light transmission rate of said membrane in a wavelength of 500nm is 90% or more. That is, the light transmission rate thereof is very high and exactly the same as that of a dish for cell culture. When said membrane is used as a cell culture substance, cell morphology can be observed easily. Even further, said membrane of the present invention has a sufficient strength and an excellent transparency, and thus is useful as an implantation material for human cornea.

Further, according to the method for preparing a non-fibrogenesis collagen material of the present invention, a collagen gel does not contain a salt in the manufacturing process. Thus, for example, a washing process to remove salt from the collagen gel is not necessary, and therefore, cost of manufacturing can be reduced. Furthermore, the transparent and non-fibrogenesis collagen membrane of the present invention has excellent properties, such as very high collagen purity.

In addition, a fish-derived collagen is used as a raw material of the non-fibrogenesis collagen material of the present invention. This is because the fish-derived collagen hardly has zoonosis. Therefore, in using the fish-derived collagen, the non-fibrogenesis collagen material can be safely used as a scaffold material for regenerative medicine, or a implantation material, compared to a collagen material prepared using collagens derived from bovine that have bovine spongiform encephalopathy (BSE), swine that have foot-and-mouth disease, or birds that have influenza infection. Further, in using the fish-derived collagen, particularly the fish scale-derived collagen, the non-fibrogenesis collagen material can have sufficient strength, despite an absence of collagen fibril formation. The non-fibrogenesis collagen material may have high strength in using the fish-derived collagen, compared to the use of the swine-derived collagen or bovine-derived collagen. The reason for this has not been fully elucidated, but is presumed to be as follows. The fish-derived collagen, in particular the fish scale-derived collagen, has a strong interaction of collagen molecules, compared to the swine- or bovine-derived collagen, and thus the non-fibrogenesis collagen material can have a high strength. However, the present invention is by no means limited to the above explanation.

BRIEF DESCRIPTION OF THE DRAWINGS

FIG. 1 is a photograph showing an appearance of the non-fibrogenesis collagen transparent membrane of the present invention.

FIG. 2 is a graph showing optical densities of the non-fibrogenesis collagen transparent membrane without and with cross-linking, at a wavelength range from 300nm to 700nm. A cell culture dish made of polystyrene is used as a control.

FIG. 3 is a graph showing the results of a tensile strength test of the non-fibrogenesis collagen transparent membrane without (o) and with (□) cross-linking.

FIG. 4 is scanning electron micrographs of the non-fibrogenesis collagen transparent membrane without cross-linking (A) and with cross-linking (B).

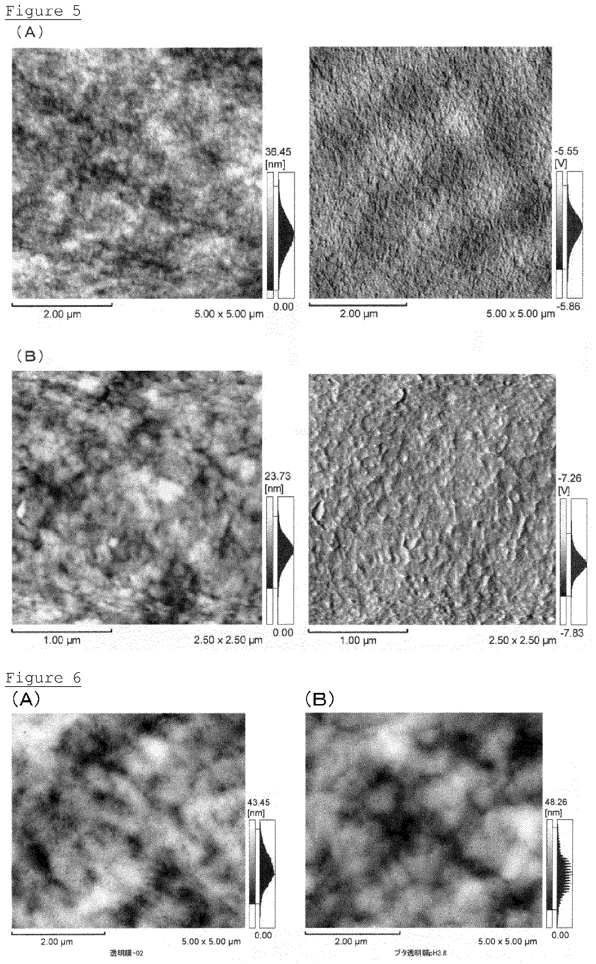

FIG. 5(A) is atomic force micrographs of the non-fibrogenesis collagen transparent membrane without cross-linking. The left micrograph is one in dynamic mode, and the right micrograph is one in phase mode. The non-fibrogenesis collagen transparent membrane has a structure wherein collagen molecules are tangled, and a surface roughness (RMS) thereof is 5.7nm. FIG. 5(B) is atomic force micrographs of the cross-linked non-fibrogenesis collagen transparent membrane. The left micrograph is one in dynamic mode, and the right micrograph is one in phase mode. The cross-linked non-fibrogenesis collagen transparent membrane has a structure wherein collagen molecules are tangled, and a surface roughness (RMS) thereof is 4.6nm.

FIG. 6 is atomic force micrographs of the non-fibrogenesis collagen transparent membrane prepared using fish scale collagen (A) or swine dermal collagen (B).

FIG. 7 is a graph showing the degree of cross-linking (A), and swelling ratios (B and C) of the non-fibrogenesis collagen transparent membrane cross-linked by glutaraldehyde evaporation.

FIG. 8 is a graph showing optical densities of the non-fibrogenesis collagen transparent membrane without cross-linking (Example 1), and with cross-linking (Examples 4 to 8) at a wavelength range from 300nm to 700nm.

FIG. 9 is a photograph showing appearances of the non-fibrogenesis collagen porous materials prepared using an acid solution containing carbon dioxide, without cross-linking (a), with thermally cross-linking (b), and with cross-linking by glutaraldehyde (c).

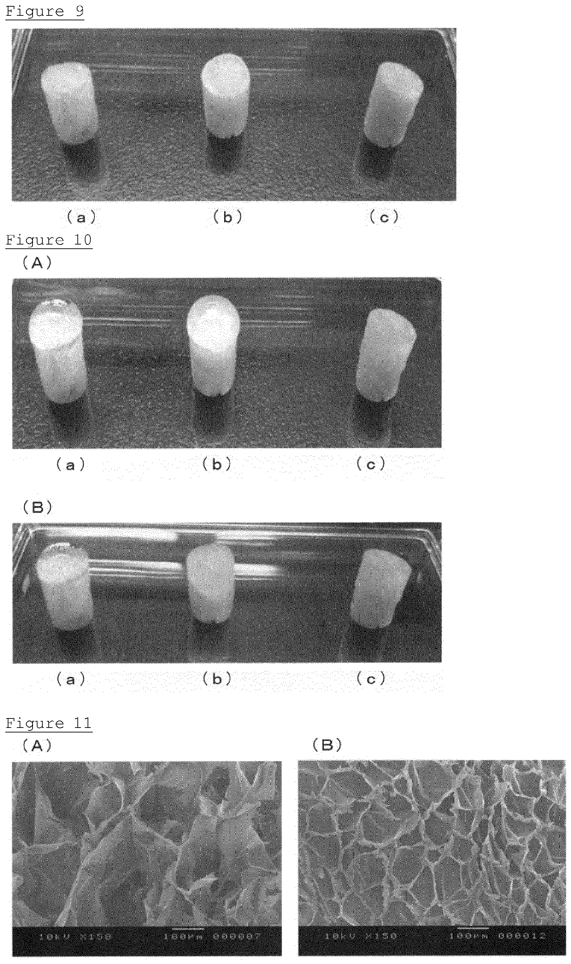

FIG. 10(A) is a photograph just after distilled water is delivered by drops into the non-fibrogenesis collagen porous material without cross-linking (a), with thermally cross-linking (b), and with cross-linking by glutaraldehyde (c). A water absorbability of the non-fibrogenesis collagen porous material was increased by glutaraldehyde cross-linking. FIG. 10(B) is a photograph 10 minutes after the distilled water is delivered by drops into the non-fibrogenesis collagen porous material without cross-linking (a), with thermally cross-linking (b), and with cross-linking by glutaraldehyde (c). FIG. 11 is scanning electron micrographs showing a structure of the non-fibrogenesis collagen porous material cross-linked by glutaraldehyde, prepared using 1% by weight of collagen solution (A), or 5% by weight of collagen solution (B). The pore diameter becomes smaller in accordance with an increase of collagen concentration.

FIG. 12 is graphs showing value of elasticity and value of viscosity of the cross-linked non-fibrogenesis collagen porous material in Example 10 (A) and Example 11 (B).

DESCRIPTION OF EMBODIMENTS

[1] Non-fibrogenesis collagen porous material

The non-fibrogenesis collagen porous material of the present invention is prepared using a fish-derived collagen.

(Collagen)

The collagen contained in the aforementioned collagen material of the present invention is the fish-derived collagen. As for the fish used to obtain the fish-derived collagen, there may be mentioned, for example, tilapia, salt-water venomous catfish, labeo rohita, catla, carp, channa argus, pirarucu, sea bream, bastard halibut, shark, salmon, and the like. However, fish living in rivers, lakes, or seas with warm water temperatures is preferable, in view of an after-mentioned denaturation temperature. In particular, examples of such fish include fish belonging to the Oreochromis genus, and tilapia in particular is preferable. The collagen obtained from fish of the Oreochromis genus has a relatively-high denaturation temperature. For example, Oreochromis niloticus, farmed in Japan or China, is easily available, and thus a large amount of collagen can be obtained therefrom.

There is no limitation to the fish parts for obtaining the collagen. As the fish part, there may be mentioned, for example, scale, skin, bone, cartilage, fin, muscle, and organ (such as air bladder), but the scale is preferable. This is because the scale carries few lipids, which induce a fishy odor. Further, the fish scale-derived collagen has an excellent cellular adhesiveness. In particular, it is considered that an interaction of collagen molecules derived from fish scales is strong, and thus, collagen material with high strength can be obtained.

Type I collagen derived from fish scale, which forms a "triple-helix structure (tropocollagen)" by assembling three polypeptide chains with a molecular weight of approximately 100 kD per polypeptide chain, has a molecular weight of approximately 300 kD. The fish scale-derived type I collagen has a stiff rod-like shape with a length of 300nm and a diameter of 1.5nm. The specific "triple-helix structure (tropocollagen)" of type I collagen derived from fish is due to an amino acid sequence of the polypeptide chain. The polypeptide chain comprises repeats of a unit of three amino acids i.e. "G-X-Y". The "G" is glycin, many of "X" are proline, and many of "Y" are hydroxyproline. Hydroxyproline is not contained in common proteins, but contained specifically in collagen. It is considered that a triple-helix structure of collagen becomes stabilized by hydrogen bonds between the hydroxyl group of hydroxyproline and hydration water. Collagen is polyamphoteric molecules with amino groups and carboxyl groups. That is, collagen charges positively in acidic solutions, and charges negatively in alkaline solutions. Further, collagen outwardly charges neutral in or around neutral pH. The collagen molecule with a triple-helix structure (tropocollagen) forms collagen fine fibril in or around neutral pH, although collagen fibril formation is strongly dependent on salt types and temperature. Each five molecules of type I collagen shifted in the direction of the long axis at 1/4 length are self-assembled to form nano fiber. Furthermore, a collagen fine fibril can be formed by assembling the nano fibers.

In a method for preparing the non-fibrogenesis collagen transparent membrane, a collagen solution does not contain salt and thus it cannot be adjusted to an ionic strength and pH for collagen fibril formation. Therefore, from the collagen solution without salt, the non-fibrogenesis collagen material can be obtained.

When the temperature is raised, the "triple-helix structure" of collagen consisting of three polypeptides untwists, and then three polypeptides come to pieces. Consequently, the collagen changes into a gelatin. Change from collagen to gelatin is referred to as denaturation. Once the denaturation of collagen occurs, it is difficult to restore the gelatin to the "triple-helix structure" even if the temperature is lowered. At best, a denaturation temperature of collagen derived from a certain living being is slightly higher than a temperature of habitat environment of a certain living being. Therefore, the denaturation temperature of collagen derived from scale of a fish which lives in the water, is not so high.

The fish-derived collagen, which may be used in the present invention, is not limited by the denaturation temperature thereof. However, it is preferable that a fish-derived collagen has a high denaturation temperature. In particular, the denaturation temperature is preferably 20°C or more, more preferably 25°C or more, even more preferably 28°C or more, most preferably 30°C or more. However, the non-fibrogenesis collagen material of the present invention can be obtained using a fish-derived collagen with a denaturation temperature at less than 20°C, by manufacturing at a temperature equal to or lower than the denaturation temperature of the fish-derived collagen.

A method for obtaining the fish-derived collagen is not particularly limited, so long as a method wherein the "triple-helix structure" of collagen may not be destroyed. For example, the fish-derived collagen can be extracted by a method disclosed in Japanese Unexamined Patent Publication (Kokai) No. 2006-257014. A method for preparing collagen from fish scales is explained below.

Firstly, the collected scales are washed in order to remove undesired substances such as fishskin and fins. If necessary, substances responsible for odor such as proteins, other than collagen, or lipids which adhere to the scale surface may be removed from scales by using alcohol such as methanol, ethanol, or isopropanol, hydrophilic organic solvent such as acetone, surfactant, salt solution such as sodium chloride solution, and alkaline solution such as sodium hydrate solution.

Next, the mineral component (calcium phosphate) included in the scales is dissolved by gently stirring with an agitating blade in a demineralizing solution for 1 to 48 hours. The demineralizing solution is not limited, so long as the mineral component can be dissolved therein. An aqueous solution of inorganic acid such as hydrochloric acid, or phosphoric acid, organic acid such as acetic acid or citric acid, ethylene diamine tetra-acetic acid, or the like, may be used. Hydrochloric acid solution or acetic acid solution, which is widely used, is preferable. The amount of demineralizing solution is not particularly limited, so long as the scales are washed with purified water after demineralization.

The scales wherein impurities are taken away, are gently stirred with an agitating blade in acidic solution containing protease for 2 to 72 hours, and whereby soluble collagen is extracted by cutting the cross-linking between collagen molecules. Subsequent steps after the above extraction step may be carried out at a temperature equal to or lower than the denaturation temperature, preferably at a temperature equal to or lower than a temperature of minus 5 degree Celsius with respect to the denaturation temperature, so that the collagen is not denatured.

Pepsin, proctase, papain, protease M, or the like, which exhibit high activity in acidic solution, is preferably used as a protease. A range of pH of the solution is not limited, so long as the activity of protease is maintained high, but generally 2 to 5. The amount of protease is not particularly limited. However, 1 to 15% by weight of protease with respect to a dry weight of the fish scales is employed conventionally. A concentration of the protease and a volume of solution may be appropriately determined for the even stirring of the scales. The used acid is not particularly limited. However, hydrochloric acid, acetic acid, citric acid, malic acid or the like, which has high safety for humans, may be preferably selected, and particularly hydrochloric acid, or acetic acid is preferabl. According to the method above, an atelocollagen, wherein nonhelical regions (telopeptides) presented on both sides of the collagen molecule are degraded, can be extracted.

The resulting soluble collagen is separated from insoluble fish scale residues via centrifugation or filtration. Soluble collagen can be extracted from the separated fish scale residues by treating the residues in acidic solution containing protease. Thus, high yield of soluble collagen may be obtained by repeating the extraction step approximately 2 to 4 times.

The resulting collagen solution contains protease, protein other than collagen, gelatin (denatured collagen), and the like. Thus, if necessary, collagen can be purified from the collagen solution. A purification method of collagen is described below. Salt such as sodium chloride is added to the soluble collagen solution. As the concentration of salt is raised, collagen is precipitated. For example, collagen can be precipitated (salt out) by adding sodium chloride to a soluble collagen solution at a final concentration of 1M and allowing it to stand for 5 minutes to 24 hours.

Collagen can be precipitated by adding sodium hydrate to the collagen solution so that the pH of the solution is raised to equal to or more than neutral pH. For example, collagen can be precipitated (salt out) by adding sodium hydrate to a soluble collagen solution at a final pH of 7 to 9 and allowing it to stand for 5 minutes to 24 hours. As the soluble collagen solution yields a white turbidity through the above procedure, precipitation of collagen may easily be confirmed.

The precipitated collagen is collected by a conventional method for separating liquids from solids, such as centrifugation or filtration, and resolved in an acidic solution by gently stirring. For example, the precipitated collagen may be gently stirred in an acidic solution of pH 2 to 4, for 1 to 48 hours. As mentioned above, collagen can be purified, and highly purified collagen can be obtained by repeating this process. Salts used in the purification step can be removed with distilled water using a dialysis membrane, etc.

[1-1] Non-fibrogenesis collagen transparent membrane

The non-fibrogenesis collagen transparent membrane has a tensile strength of 30MPa or more, and a density determined by the gravimetric method of 0.4g/cm$^3$ or more.

(Tensile strength)

The tensile strength of the non-fibrogenesis collagen transparent membrane of the present invention is 30MPa or more, preferably 40MPa or more, more preferably 50MPa or more. A sufficient tensile strength cannot be obtained if it is less than 30MPa, which is normally a sufficient strength when used as a biomaterial. Further, when the non-fibrogenesis collagen transparent membrane is cross-linked, the tensile strength of the non-fibrogenesis collagen transparent membrane of the present invention may be 50MPa or more. That is, the tensile strength of the cross-linked non-fibrogenesis collagen transparent membrane is preferably 60MPa or more, more preferably 70MPa or more, most preferably 80MPa or more.

There is no limitation to the upper limit of the tensile strength of the membrane, but preferably 200MPa or less, more preferably 150MPa or less, most preferably 120MPa or less. When the tensile strength is more than 200MPa, the non-fibrogenesis collagen transparent membrane, in implanting it, may sometimes not bind tissue therearound.

A conventional method can be used for a tensile strength test. That is, both sides of a specimen having a width of 10mm and a length of 20 to 30mm are fixed on a tensile tester so that the distance between load cells is 10mm. The specimen is pulled at a rate of 0.5mm/minute, and strain (%) at fracture and stress (g) at fracture are measured by a tensile tester (Orientec;STA-1150). The average values of five specimens are measured. Further, a specimen thickness is measured with a micrometer, and the tensile strength of the specimen is calculated.

(Density)

The non-fibrogenesis collagen transparent membrane of the present invention has a density of 0.4g/cm$^3$ or more, preferably 0.5g/cm$^3$ or more, more preferably 0.6g/cm$^3$ or more, even more preferably 0.65g/cm$^3$ or more, most preferably 0.8g/cm$^3$ or more, determined by the gravimetric method. When the density is less than 0.4g/cm$^3$, mechanical strength of the non-fibrogenesis collagen transparent membrane is deficient. There is no particular upper limit for the density, but it is preferably 1.2g/cm$^3$ or less, more preferably 1.15g/cm$^3$ or less, most preferably 1.1g/cm$^3$ or less. When the density is more than 1.2g/cm$^3$, impurities may cause contamination to the non-fibrogenesis collagen transparent membrane in the drying step. The density determined by the gravimetric method can be calculated by dividing weight by the volume of the non-fibrogenesis collagen transparent membrane.

(Surface roughness)

The surface roughness of the non-fibrogenesis transparent collagen membrane is extremely low, and preferably 30nm or less, more preferably 20nm or less, most preferably 10nm or less. When the surface roughness is more than 30nm, collagen fibrils formed from collagen molecules may be contained in the membrane. The low surface roughness means that the non-fibrogenesis collagen transparent membrane of the present invention is not collagen fibers membrane but non-fibrogenesis collagen membrane composed of collagen molecules. That is to say, the surface of the non-fibrogenesis collagen transparent membrane of the present invention is smooth due to a lack of collagen fine fibrils that appear an irregular structure on the surface thereof. The smooth surface of the non-fibrogenesis collagen transparent membrane can not scatter light and shows an excellent transmittance.

The surface roughness can be measured by a commercially available cantilever and atomic force microscope in accordance with the following method. A tapping mode image is measured at the scanning range of $4\mu m^2$ and at a constant rateof 0.7Hz to 1.2Hz. The roughness of plane (surface roughness) can be calculated using software supplied with the atomic force microscope.

(Light transmission rate)

The light transmission rate (degree of transparency) of the non-fibrogenesis collagen transparent membrane in the present invention is extremely high, for example it is closer to that of a polystyrene plate for cell cultures. The light transmission rate of the non-fibrogenesis collagen transparent membrane in the present invention may be 90% or more at any wavelength among 480nm to 700nm, but preferably 90% or more at wavelengths ranging from 500nm to 700nm, more preferably 90% or more at wavelengths ranging from 480nm to 700nm. Further, the light transmission rate of the non-fibrogenesis collagen transparent membrane without cross-linking is most preferably 90% or more at wavelengths ranging from 400nm to 700nm.

There is no limitation to the thickness of the non-fibrogenesis collgen transparent membrane for measuring the light transmission rate, but the rate is 90% or more in respect with the thickness of $1\mu m$ to 1mm.

The light transmission rate can be measured in accordance with the following method. The non-fibrogenesis collagen transparent membrane is attached to a cell culture dish (polystyrene dish), and then, for example, the light transmission rate can be measured to scan at a wavelength range of 300nm to 700nm by using the POWERSCAN HT (DS Pharma Biomedical Co., Ltd). The light transmission rate can be calculated by the following formula using absorbance (O.D.): Light transmission rate = $1/10^{O.D.} \times 100$

There is no limit to the thickness of the non-fibrogenesis collagen transparent membrane, but it is preferably $1\mu m$ to 1mm, more preferably $2\mu m$ to $500\mu m$, most preferably $3\mu m$ to $100\mu m$. When the membrane thickness is less than $1\mu m$, the membrane may tear during the drying step. When the membrane thickness is more than 1mm, the surface smoothness of the membrane may be reduced. Further, there is no limitation to the variation in membrane thickness of the non-fibrogenesis transparent collagen membrane, but it is preferably plus or minus 40%, more preferably plus or minus 35%, even more preferably plus or minus 30% relative to the average membrane thickness. When the variation in membrane thickness is more than 40%, a part with high strength and a part with low strength are present in the non-fibrogenesis collagen transparent membrane, and thus, the mechanical strength, such as tensile strength, may sometimes be decreased.

(Structure of non-fibrogenesis transparent collagen membrane)

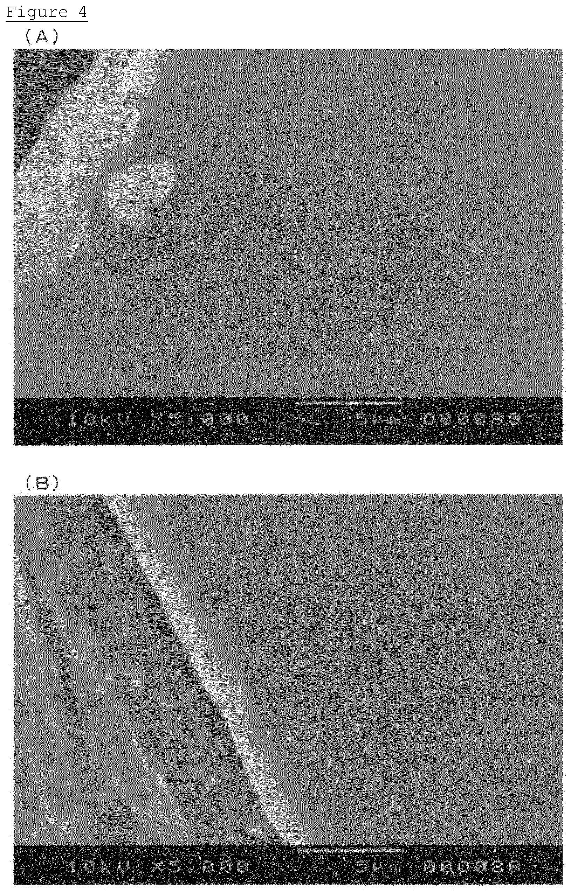

As shown in Figure 4, the non-fibrogenesis collagen transparent membrane of the present invention does not contain collagen fine fibrils. That is to say, it is not subject to fibrogenesis. Further, as shown in Figure 5, the non-fibrogenesis collagen transparent membrane of the present invention contains collagen molecules (tropocollagen) and exhibits high surface smoothness. In the non-fibrogenesis collagen transparent membrane, collagen molecules are densely packed, and thus the non-fibrogenesis collagen transparent membrane has a high tensile strength of 30MPa or more. Further, cross-linking as described later can increase the tensile strength of the membrane.

(Cross-linking)

The non-fibrogenesis collagen transparent membrane of the present invention may be cross-linked. Cross-linking can further increase the mechanical characteristics of the collagen fiber membrane. The non-fibrogenesis collagen transparent membrane does not contain the collagen microfibrils. It is considered that the collagen molecules (triple-helix structure of collagen) are cross-linked. The tensile strength of the non-fibrogenesis collagen transparent membrane is surprisingly increased thanks to the cross-linking of the collagen molecules. Specifically, the tensile strength of the cross-linked non-fibrogenesis collagen transparent membrane is 70MPa or more, preferably 80MPa or more, more preferably 90MPa or more. The upper limit of the tensile strength of the cross-linked non-fibrogenesis collagen transparent membrane is not particularly limited, but preferably 200MPa or less, more preferably 150MPa or less, most preferably 120MPa or less. When the tensile strength is more than 200MPa, the membrane, in implanting it, sometimes may not bind tissues therearound.

(Degree of cross-linking)

The level of cross-linking of the non-fibrogenesis collagen transparent membrane can be defined by a degree of cross-linking. A method for defining said degree is not limited. For example, when a non-fibrogenesis collagen transparent membrane is cross-linked by glutaraldehyde, amino groups of the collagen are expended. Thus, the degree of cross-linking can be measured by quantifying free amino groups of the collagen fiber membrane. Specifically, an amount of free amino groups are measured by a TNBS method using trinitrobenzene sulfonate.

There is no particular limitation to the degree of cross-linking of a non-fibrogenesis collagen transparent membrane. However, the lower limit of the degree of cross-linking is preferably 5% or more, more preferably 15% or more, most preferably 30% or more. The upper limit of the degree of cross-linking is preferably 90% or less, more preferably 80% or less, most preferably 75% or less. When the degree of cross-linking is less than 5%, the non-fibrogenesis collagen transparent membrane is easily degraded by proteases. When the degree of cross-linking is more than 90%, the non-fibrogenesis collagen transparent membrane is hardly degradable in vivo.

(Swelling ratio)

The non-fibrogenesis collagen transparent membrane of the present invention has a high swelling ratio, and thus the non-fibrogenesis collagen transparent membrane shows resistant to Dulbecco's phosphate buffered saline (calcium and magnesium free).

There is no particular limitation to the swelling ratio of the non-fibrogenesis collagen transparent membrane. However, the upper limit of the swelling ratio is preferably 600% or less, more preferably 500% or less. The lower limit of the swelling ratio is 100% or more. The term "swelling ratio of 100%", as used herein, means that the non-fibrogenesis collagen transparent membrane does not swell at all. When the swelling ratio is more than 600%, the collagen molecules are dispersed.

[1-2] Non-fibrogenesis collagen porous material, which is not claimed.

The non-fibrogenesis collagen porous material of the present invention has 80% or more porosity. The porosity is more preferably 85% or more, most preferably 90% or more. When the porosity is 80% or more, cells or tissues can easily penetrate into the non-fibrogenesis collagen porous material. The non-fibrogenesis collagen porous material of the present invention can obtain high porosity without collagen fibril formation. Thus, the non-fibrogenesis collagen porous material can be used as a cell culture substrate, a scaffold material for regenerative medicine (material for tissue engineering of cartilage, bone, ligament, corneal stroma, skin, or liver), an implantation material (for example, wound dressing material, bone grafting material, hemostatic material, or anti-adhesive material, for example) or a carrier for drug delivery.

(Water permeability)

Preferably, the non-fibrogenesis collagen porous material of the present invention has appropriate water permeability. When cells are cultured on the non-fibrogenesis collagen porous material, the water permeability is important as an index of a cell penetration into said material. As shown in Example 9, compared to the non-fibrogenesis collagen porous material without cross-linking (a), and the non-fibrogenesis collagen porous material cross-linked by thermal dehydration (b), the non-fibrogenesis collagen porous material cross-linked by glutaraldehyde (c) has good water absorbability, and therefore, is preferable as a cell culture application.

(Viscoelastic property)

A value of elasticity of the non-fibrogenesis collagen porous material of the present invention is not limited, but preferably 2.0kPa to 10MPa, more preferably 20kPa to 2MPa. When the value of elasticity is 2.0kPa to 10MPa, the cells harvested can easily penetrate into the non-fibrogenesis collagen porous material, and further, said material's ease of handling is excellent.

The value of viscosity of the non-fibrogenesis collagen porous material of the present invention is not limited, but preferably 1.0 to 500kPa, more preferably 1.5 to 100kPa. When the value of viscosity is 1.0 to 500kPa, the cells harvested can easily penetrate into the non-fibrogenesis collagen porous material, and further, said material's ease of handling is excellent.

(Average pore diameter)

There is no particular limitation to an average pore diameter of the non-fibrogenesis collagen porous material of the present invention, but preferably 50$\mu$m to 500$\mu$m, more preferably 80$\mu$m to 300$\mu$m. When the average pore diameter is 50$\mu$m to 500$\mu$m, cells or tissues can easily penetrate into the material, and thus said material is the most appropriate as a cell culture substrate or an implantation material.

The average pore diameter of the non-fibrogenesis collagen porous material can be measured by a mercury press-injection method or an observation with a scanning electron microscope. The following is the method for measuring average pore diameter via observation with a scanning electron microscope. A cross-section at any height is prepared from the non-fibrogenesis collagen porous material obtained. Then, to prevent a static charge, a platinum coating of 20nm or less or the like is applied to the cross-section. The observation is carried out at an accelerate voltage of 10kV or less. The section is observed preferably at 1000 fold or less of magnification and the pore diameters of 100 or more are measured. The average pore diameter can be measured by obtaining a distribution of the pore diameters and calculating the average of the pore diameters.

[2] Method for preparing non-fibrogenesis collagen material

A method for preparing non-fibrogenesis collagen material of the present invention comprises the following steps: (1) preparing a collagen acid solution containing carbon dioxide and collagen (hereinafter, referred to as a collagen acid solution preparation step) and (2) forming a collagen material from the collagen acid solution (hereinafter, referred to as a collagen forming step). In the method for preparing non-fibrogenesis collagen material of the present invention, the non-fibrogenesis collagen material may basically be prepared in accordance with conventional methods for preparing collagen material, except for the use of the collagen acid solution.

(Collagen)

A collagen used in the method for preparing non-fibrogenesis collagen material is not limited. For example, the collagen described in the above section "[1] Non-fibrogenesis collagen porous material" may be used.

(Collagen acid solution)

The collagen acid solution may be prepared, for example, by a method for bubbling carbon dioxide in aqueous solvent or a method for adding dry ice to the aqueous solvent. There is no particular limitation to the amount of dissolution of carbon dioxide. However, in order to dissolve the collagen, the pH of the solvent is preferably pH 2.0 to 4.0. Therefore, it is preferred that carbon dioxide is dissolved in the solvent so that the pH of the solvent is pH 4.0 or less.

(Material)

A material prepared through the method for preparing non-fibrogenesis collagen material of the present invention is not particularly limited, so long as the material contains collagen, there may be mentioned a collagen membrane or a collagen porous material, as the collagen material. A form of the collagen material is also not particularly limited, but there may be mentioned, for example, film, sheet, or sponge, as the form of collagen material.

(Collagen acid solution preparation step)

In the collagen acid solution preparation step, for example, (1) the carbon dioxide acid solution may be prepared by dissolving carbon dioxide in a solvent, and then the collagen acid solution may be prepared by dissolving collagen in the carbon dioxide acid solution. Alternatively, (2) the collagen acid solution may be prepared by dissolving carbon dioxide and collagen in a solvent simultaneously.

(Collagen forming step)

In the collagen forming step, the collagen acid solution can be poured into a forming mold and dried, and thereby obtain the collagen material without collagen fibril formation.

(Drying step)

In the drying step, the solvent is removed from the material and the material is dried. In this step, a density of the material may be increased and the mechanical strength thereof can be increased. A method for drying collagen material is not limited, as long as the method is not carried out at a temperature equal to or higher than the denaturation temperature, at which the triple-helix structure of collagen is destroyed. For example, a lyophilization method, cast method, air drying method, or air seasoning method may be used. In the case of the lyophilization method, the resulting collagen gel is frozen by rapidly, constantly, or gradually, reducing the temperature from 0°C to -80°C. A liquid sublimes from the frozen collagen gel under vacuum state, and thereby obtains a dried non-fibrogenesis collagen porous material.

(Cross-linking step)

The method for preparing non-fibrogenesis collagen material may further comprise a step of cross-linking the dried collagen material. In the cross-linking step, collagen molecules are cross-linked with one another.
Regarding the cross-linking procedure, the conventional process may be used, i.e., physical cross-linking or chemical cross-linking. Physical cross-linking includes thermal cross-linking (cross-linking by thermal dehydration;DHT), cross-linking via ultraviolet (UV) radiation, or cross-linking via gamma radiation. In the thermal cross-linking, the collagen fiber membrane is allowed to stand at 100°C to 140°C for 1 to 48 hours in a vacuum. However, the high-temperature treatment may denature the collagen. Therefore, chemical cross-linking carried out at a temperature equal to or lower than the denaturation temperature is preferable.
As the cross-linking agent used in chemical cross-linking, chemical cross-linkers such as glutaraldehyde (GA), polyepoxy compound, carbodiimide, isocyanate, or genipin, can be used. In chemical cross-linking, for example, the fish scale-derived, non-fibrogenesis collagen material is cross-linked by immersing said membrane in a solvent containing the cross-linker. In the case of glutaraldehyde, for example, the non-fibrogenesis collagen material can be uniformly cross-linked in a glutaraldehyde solution of 0.5% to 2.0% by weight. Preferably, however, the non-fibrogenesis collagen material is treated at approximately 20°C to 40°C for 1 to 24 hours under reduced pressure, in a desiccator including a 25% glutaraldehyde solution or a diluted solution thereof. An inner part of the non-fibrogenesis collagen material can also be uniformly cross-linked through glutaraldehyde evaporation. This cross-linking increases the strength of the non-fibrogenesis collagen material.
A method for preparing a non-fibrogenesis collagen transparent membrane, which is one of the non-fibrogenesis collagen materials of the present invention, will be described hereinafter.

[2-1] Method for preparing a non-fibrogenesis transparent collagen membrane

The method for preparing the non-fibrogenesis collagen transparent membrane of the present invention comprises the following steps: (1) dissolving a collagen in an acid solution (hereinafter, referred to as a collagen dissolving step), (2) pouring the collagen solution into a forming mold to obtain a collagen membrane (hereinafter, referred to as a collagen forming step), and (3) drying the collagen membrane (hereinafter, referred to as a solvent removal and drying step).

According to the method for preparing the non-fibrogenesis collagen transparent membrane of the present invention, the non-fibrogenesis collagen transparent membrane of the present invention can be manufactured. However, the non-fibrogenesis collagen transparent membrane can be manufactured by methods other than said method of the present invention.

(Collagen)

As a collagen used in the method for preparing a non-fibrogenesis collagen transparent material, the collagen described in the above section "[1], Non-fibrogenesis collagen porous material" may be used.

(Collagen dissolving step)

In the collagen dissolving step, collagen is dissolved in an acid solution.
The term "aqueous solvent", as used herein, means a solvent wherein water and an organic solvent are mixed. The organic solvent is not limited, so long as it may be mixed with water, and the collagen may be dissolved in the mixture, but with preferably lower alcohol. For example, lower alcohol having 1 to 4 carbon atoms i.e. methanol, ethanol, n-propyl alcohol, isopropyl alcohol, n-butyl alcohol, isobutyl alcohol, sec-butyl alcohol, or tert-butyl alcohol, can be used as the lower alcohol. Such lower alcohol is relatively inexpensive for a manufacturers' cost.
The acid solution may be prepared, for example, by a method for bubbling carbon dioxide in an aqueous solvent or a method for adding a dry ice to an aqueous solvent. There is no particular limitation to an amount of dissolution of carbon dioxide, however, in order to dissolve the collagen, the pH of the solvent is preferably pH 2.0 to 4.0. Therefore, it is preferable that carbon dioxide is dissolved in the solvent so that the pH of the solvent is pH 4.0 or less.
The collagen is dissolved in the prepared acid solution, and thereby a collagen solution can be obtained. Collagen can be dissolved in the above acid solution as adjusted, and thus, for example, 5% by weight or more of a collagen solution with high viscosity is obtainable.

(Collagen forming step)

In the collagen forming step, the solubilized collagen solution is poured into a forming mold in order to obtain a collagen membrane.

(Water removal and drying step)

In the water removal and drying step, the aqueous solvent is removed and the collagen membrane is dried. In the water removal and drying step, there is an increase in the density of the collagen molecules, and the strength of the collagen transparent membrane may also increase.
The water removal and drying are carried out by evaporating the aqueous solvent from the upper surface of the collagen membrane gel. Alternatively, the lower surface and the upper surface of the collagen membrane gel are covered by a flat plate capable of preventing aqueous solvent transmission, and the aqueous solvent can be gently removed only from a side of the collagen fiber gel. By covering the gel with the flat plate, the non-fibrogenesis collagen transparent membrane having an equalized membrane thickness may be obtained, and thus the mechanical strength of the non-fibrogenesis collagen transparent membrane can be increased. There is no limitation to the material of the plate, but there may be mentioned polystyrene, silicone, polyester, polyamide, polypropylene, polyethylene, polymethylmethacrylate or glass, as the material of the plate. Specifically, polystyrene is preferable because it exhibits an excellent dissociating property from the non-fibrogenesis collagen transparent membrane.
Time for removing water and drying is not particularly limited, so long as the aqueous solvent of 90% or more may be removed, but preferably 3 hours to 14 days, more preferably 5 hours to 7 days, most preferably 12 hours to 3 days.

(Cross-linking step)

Further, a cross-linking step of the non-fibrogenesis collagen transparent membrane may be included in the method for preparing the non-fibrogenesis collagen transparent membrane of the present invention. The non-fibrogenesis collagen transparent membrane does not contain collagen fine fibrils, therefore, the collagen molecules (tropocollagen) in the non-fibrogenesis collagen transparent membrane may be cross-linked with one another.

Regarding cross-linking procedure, a conventional procedure such as physical cross-linking or chemical cross-linking, may be used. The physical cross-linking includes thermal cross-linking (cross-linking by thermal dehydration;DHT), cross-linking using ultraviolet (UV) radiation, or cross-linking using gamma radiation. In the thermal cross-linking, the non-fibrogenesis collagen transparent membrane is allowed to stand at 100°C to 140°C for 1 to 12 hours in a vacuum. However, the high-temperature treatment may denature the collagen. Therefore, chemical cross-linking carried out at a temperature equal to or lower than the denaturation temperature is preferable.

As the cross-linking agent used in chemical cross-linking, a chemical cross-linking agent such as glutaraldehyde (GA), polyepoxy compound, carbodiimide, isocyanate, or genipin, can be used. In chemical cross-linking, for example, the non-fibrogenesis collagen transparent material can be cross-linked by immersing said membrane in a solvent containing the cross-linking agent. In the case of glutaraldehyde, for example, the non-fibrogenesis collagen transparent material can be uniformly cross-linked in a glutaraldehyde solution of 0.5% to 2.0% by weight. However, swelling may sometimes occur in the membrane when it is immersed in solvent. Therefore, preferably, the non-fibrogenesis collagen transparent material is cross-linked by using the cross-linking agent evaporation. For example, the non-fibrogenesis collagen transparent membrane is treated at approximately 20°C to 40°C for 1 to 24 hours under reduced pressure, in a desiccator including a 25% glutaraldehyde solution or a diluted solution thereof. The strength of the non-fibrogenesis collagen transparent membrane is increased through cross-linking.

EXAMPLES

The present invention now will be further illustrated by, but is by no means limited to, the following Examples.

<<Preparing Example 1: Preparation of fish-derived collagen >>

A method for preparing tilapia scale collagen will be described hereinafter.

The tilapia scales were carefully washed with water, and further carefully washed in a 10% sodium chloride solution. Impurities such as fins were removed during washing, and then the tilapia scales were dried at room temperature. A water content ratio of the tilapia scales was 18.5% by weight. 1 kg of tilapia scales was dispersed in 9 kg of hydrochloric acid solution at pH 2, and the solution including the scales was gently stirred at 25 °C, while maintaining a pH 2 by adding a 1M hydrochloric acid solution. Through this treatment, mineral elements included in the scales were disolved in the hydrochloric acid solution. The scales were collected by mesh, and then fully washed with purified water. Then, the hydrochloric acid solution at pH 2 was added to the scales so that the total weight of the whole was 4 kg.

Then, 24g of pepsin (Wako Pure Chemical Industries, Co., Ltd.; 1:10000) was added to the whole, and the whole was gently stirred at 25°C for 24 hours, using an agitating blade, whereby collagen was dissolved into the hydrochloric acid solution from the scales. A soluble collagen solution was separated from scale residue by mesh, and then the supernatant was collected through centrifugation (10000G, for 60 minutes) so that the soluble collagen solution was separated from the fine scale residue. 0.5g of pepsin was added to the soluble collagen solution and the mixture was allowed to stand at 25°C for 24 hours.

The resulting solution (2.4kg) was filtered with $0.45\mu m$ of a membrane filter. Then, sodium chloride was added to give 1.0 M of the final concentration, and the whole was gently stirred. After salting-out at 25°C for 30min, the supernatant was removed through centrifugation (10000G, for 20 minutes), and the precipitate of collagen was collected. 400mL of hydrochloric acid solution at pH 3 was added to the precipitate, and the collagen was dissolved in the solution by gently stirring at 5°C for 24 hours. This purification step of salting-out was repeated three times, and the hydrochloric acid solution containing the tilapia scale collagen was obtained. The resulting hydrochloric acid solution containing the tilapia scale collagen was dialyzed with distilled water, and lyophilized.

«EXAMPLE 1»

In this Example, the non-fibrogenesis collagen transparent membrane was prepared from a collagen solution with a 1mm thickness.

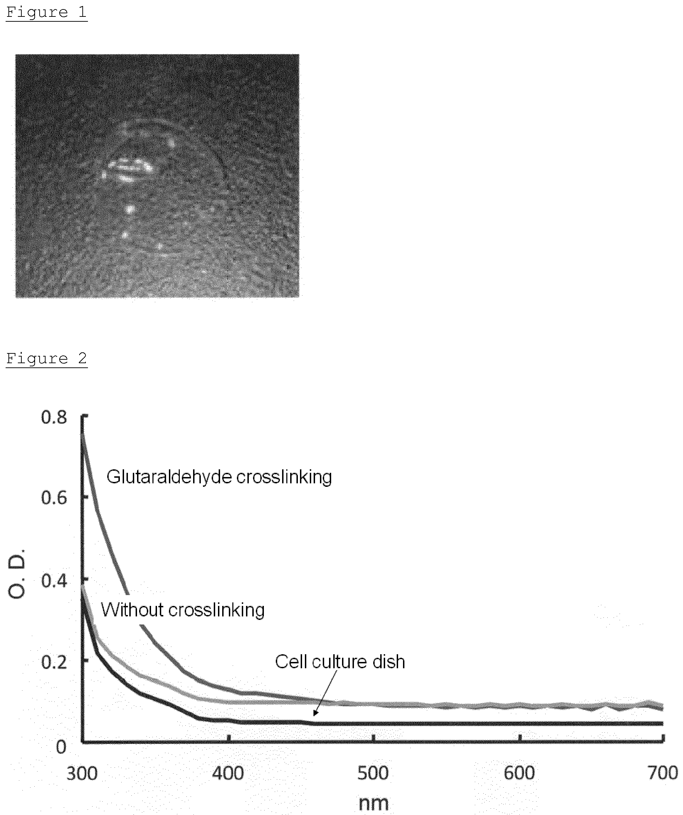

An acid solution at pH 3.0 was prepared by bubbling carbon dioxide in distilled water. The lyophilized collagen obtained in Preparing Example 1 was dissolved in the resulting acid solution so that the concentration became 1% by weight, in order to obtain a collagen solution. A silicone plate with a 1mm thickness and a cylindrical hole of 18mm in diameter was created, and the lower surface was covered by a silicone plate, to obtain a forming mold. The 1% by weight of the collagen solution was added dropwise to the cylindrical hole so that a height of the collagen solution became 1mm, and dried over night at 28°C. A volume of the collagen solution before drying was 0.254cm$^3$, and a weight of collagen contained therein was 0.254mg. A thickness of the dried non-fibrogenesis collagen transparent membrane was 8.7$\pm$4$\mu$m. Therefore, the density of the resulting non-fibrogenesis collagen transparent membrane was 1.034g/cm$^3$. The appearance thereof is shown in Figure 1.

«EXAMPLE 2»

In this Example, the cross-linked non-fibrogenesis collagen transparent membrane was prepared from the collagen solution with a 1mm thickness.

The non-fibrogenesis collagen transparent membrane obtained in Example 1 and a 25% by weight of glutaraldehyde solution ware placed in a desiccator. The desiccator was vacuated and glutaraldehyde was evaporated. The non-fibrogenesis collagen transparent membrane was allowed to stand at 37°C for 24 hours, and then was chemically cross-liked.

<<Measurement of light transmission rate>>

A light transmission rate of the non-fibrogenesis collagen transparent membrane prepared in Example 1 and the cross-linked non-fibrogenesis collagen transparent membrane prepared in Example 2, were measured. Each of the non-fibrogenesis collagen transparent membranes was attached to a cell culture dish (polystyrene dish) using tweezers, and scanned at a wavelength range of 300nm to 700nm using the POWERSCAN HT (DS Pharma Biomedical Co., Ltd).

The results are shown in Figure 2. The non-fibrogenesis collagen transparent membrane prepared in Example 1 has a transparency similar to that of the cell culture dish. The cross-linked non-fibrogenesis collagen transparent membrane prepared in Example 2 showed a slightly reduced transparency at wavelengths of less than 480nm, due to glutaraldehyde. However, the cross-linkednon-fibrogenesis collagen transparent membrane has a light transmission rate of 90% or more at wavelengths of 480nm or more. The light transmission rate was calculated by the following formula using absorbance (O.D.):

$$\texttt{Light transmission rate = 1/10\^{}O.D.×100}$$

The light transmission rate of the non-fibrogenesis collagen transparent membrane was calculated by subtracting an absorbance of the polystyrene dish from an absorbance of the non-fibrogenesis collagen transparent membrane.

<<Tensile strength test>>

The tensile strength test was carried out.

The procedures of Examples 1 and 2 were repeated, except that the polystyrene mold having a width of 45mm, a length of 70mm and a height of 1mm was used in place of the silicone container having a diameter of 18mm and a height of 5mm, to obtain the non-fibrogenesis collagen transparent membrane and the cross-linked non-fibrogenesis collagen transparent membrane.

For the tensile strength test, in order to obtain the strip specimens with a width of 10mm and a length of 20 to 30mm, the resulting collagen membranes were cut off. Both membrane thicknesses measured by a micrometer were 8.7$\pm$0.4$\mu$m.

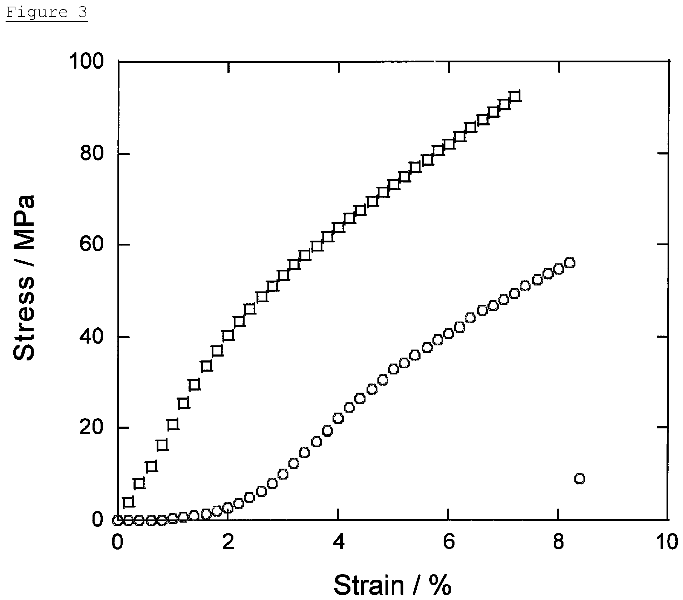

Both sides of the specimen were fixed on glass and a tensile strength test was carried out using the tensile tester (Orientec; STA-1150). The distance between load cells was 10mm, and the specimen was pulled at a rate of 0.5mm/minute. Five specimens were measured and the average thereof was calculated. The results are shown in Figure 3. In the non-fibrogenesis collagen transparent membrane without cross-linking, early increases of stress was few. That is, the stress began to increase at a strain of about 2%, and the specimen was fractured at a strain of about 8%. The maximum stress was 56MPa. While in the cross-linked non-fibrogenesis collagen transparent membrane, early increases of stress were large, and the specimen was fractured at a strain of about 7%. The maximum stress was high, i.e. 92MPa. It was found that both the strength and Young's modulus increased by cross-linking.

<<Structure of collagen membrane (surface roughness)>>

Scanning electron micrographs of the surface of the non-fibrogenesis collagen transparent membranes prepared in Examples 1 and 2 were taken. The micrographs are shown in Figure 4(A) and Figure 4(B), respectively. The non-fibrogenesis collagen transparent membranes were coated with 30nm of gold, and the surface thereof was observed with a scanning electron microscope. A collagen fiber was not observed on the surface. Further, the structure of the surface was not changed by chemical cross-linking.

Additionally, atomic force micrographs of the non-fibrogenesis collagen transparent membranes prepared in Examples 1 and 2 were taken. The micrographs are shown in Figure 5(A) and Figure 5(B), respectively. Although collagen structures were not observed in the scanning electron micrographs, collagen molecules were observed in the atomic force micrographs.

Further, the surface roughness was calculated using software supplied with the atomic force microscope. As a result, surface roughness (RMS) in the observed regions of the membranes was 5.7nm or 4.6nm respectively, which are quite low. These results indicate that the non-fibrogenesis collagen transparent membrane of the present invention is not the collagen fiber membrane but rather the non-fibrogenesis collagen membrane consisting of collagen molecules.

«EXAMPLE 3»

The procedures of Example 1 were repeated, except that pH 3.8 of the acid solution was used instead of pH 3.0 of the acid solution, and a collagen solution with a 2.5mm thickness was used instead of the collagen solution with a 1mm thickness, to obtain a non-fibrogenesis collagen transparent membrane.

«COMPARATIVE EXAMPLE 1»

The procedures of Example 3 were repeated, except that the porcine dermis-derived type I collagen (Nitta Gelatin Inc.,) was used instead of the fish-derived collagen obtained in Preparing Example 1, to obtain a non-fibrogenesis collagen transparent membrane.

<<Tensile strength test>>

The tensile strength test of the non-fibrogenesis collagen transparent membranes obtained in Example 3 and Comparative Example 1 were carried out. The membrane thicknesses of the obtained membranes were measured by a micrometer, with both thicknesses being $25 \pm 5 \mu m$. Both sides of the specimen were clipped by fixture and a tensile strength test was carried out using the tensile tester (Orientec; STA-1150). The distance between load cells was 10mm, and the specimen was pulled at a rate of 0.5mm/minute. Five specimens were measured and the average thereof was calculated.

The membrane prepared using the fish scale-derived collagen had a 1.32 times higher tensile strength compared with that prepared using the porcine dermis-derived collagen. Figure 6 shows the appearances of the non-fibrogenesis collagen transparent membranes observed by an atomic force microscope. The non-fibrogenesis collagen transparent membranes were prepared using the fish scale-derived collagen (A) and porcine dermis-derived collagen (B). There is no significant structural difference between both membranes, but it was found that the membrane prepared using the fish scale-derived collagen is composed of smaller domains.

<<EXAMPLES 4 to 8>>

In these Examples, the non-fibrogenesis collagen transparent membranes obtained in Example 1 were chemically cross-linked for different times, using glutaraldehyde evaporation.

20 mL of 10% glutaraldehyde solution and the resulting transparent membrane were placed on a mesh in a desiccator, and the transparent membrane was allowed to stand at 37 °C under reduced pressure. The membranes were treated for 15 min (Example 4), 30 min (Example 5), 1 hour (Example 6), 2 hours (Example 7), or 3 hours (Example 8), respectively. Glutaraldehyde was evaporated in the above procedures, and collagen molecules in the non-fibrogenesis collagen transparent membrane can be cross-linked. When the treatment time is longer, the membrane turns to bister.

<<Measurement of the degree of cross-linking>>

The degree of cross-linking of the resulting cross-linked non-fibrogenesis collagen transparent membrane was measured. The degree of cross-linking was quantified by the measurement of the amount of free amino groups by means of the TNBS method using trinitrobenzene sulfonate.

10mg of a specimen was weighed from each collagen transparent membrane prepared in Examples 4 to 8. 1.0mL of sodium hydrogen carbonate(4%(w/v))/TNBS(0.5%(w/v)) solution was added to the specimen, and the specimen was treated at 40°C for 2 hours. 3mL of hydrochloric acid (6N) was further added thereto, with the whole treated for 20 to 40 min at 40°C in a water bath. After hydrolyzing, 15mL of purified water was added to the whole. 1mL of solution was collected, cooled to room temperature, and diluted using 5mL of purified water. Then, the absorbance, at a wavelength of 345nm of the resulting solution, was measured. The amount of free amino groups was calculated using the following formula:

$$\text{Amount of free amino groups } (A_{g\text{-}col}; mol)/\text{amount of collagen}$$
$$(g) = (4 \times \text{absorbance})/(1.46 \times 106 (L/mol \cdot cm) \cdot \text{length of cell } (cm))$$

The amount of free amino groups ($A_{col}$) was measured by the TNBS method using untreated collagen. The degree of cross-linking (D;%) was calculated using the following formula:

$$(1 - A_{g\text{-}col}/A_{col}) \times 100$$

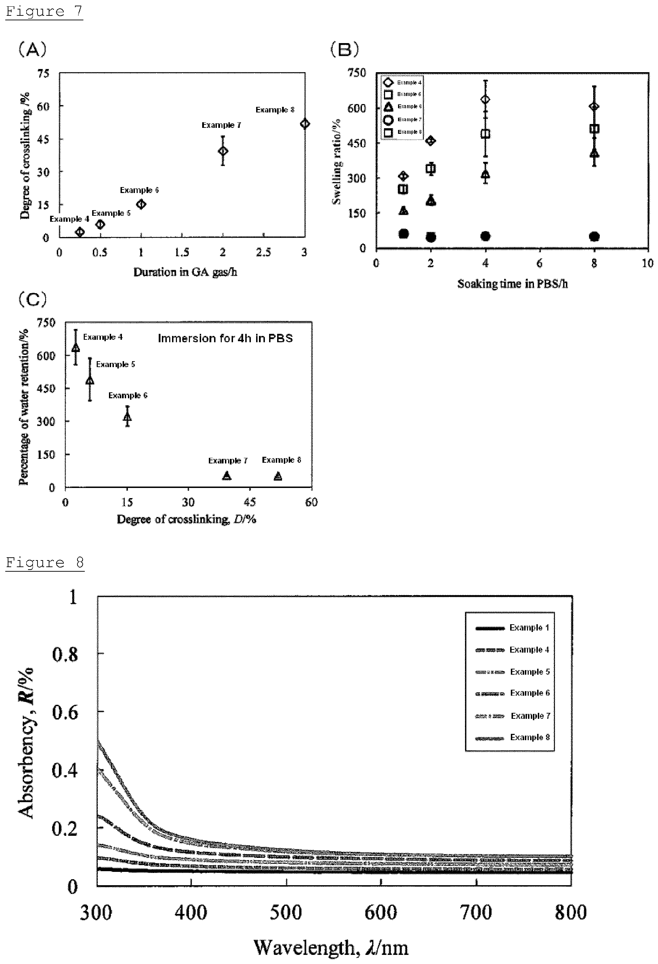

The relationship between the treatment time of cross-linking and the degree of cross-linking is shown in Figure 7A. The degree of cross-linking was increased, in a linear manner, in proportion to the treatment time of cross-linking as shown in Figure 7A.

«Swelling ratio»

A measurement was taken for the swelling ratio of the resulting non-fibrogenesis collagen transparent membrane Said measurement was carried out as follows.

Each collagen transparent membrane prepared in Examples 4 to 8 was immersed in Dulbecco's PBS for 1 hour, 2 hours, 4 hours, or 8 hours at 38°C. Then, the collagen transparent membrane was collected from Dulbecco's PBS, and an excess fluid was wiped from the membrane with a paper towel (Kimwipe). The swelling ratio was measured by weight changes before and after treatment. Said ratio (%) was calculated using the following formula:

$$(W_{PBS} - W_{DRY})/W_{DRY} \times 100$$

Variation of the swelling ratio, according to the immersion time, is shown in Figure 7B. In the transparent membranes that were cross-linked for 1 hour or less, the swelling ratio was increased by immersion for 4 hours or less, in a linear manner. In the transparent membranes, which were cross-linked for 2 hours or more, the swelling ratio remained unchanged by the immersion for 1 hour or more. Further, there was a negative correlation between the degree of cross-linking and the swelling ratios after 4 hours of immersion (Figure 7C).

<< Light transmission rate (Absorbance) >>

The chemically cross-linked non-fibrogenesis collagen transparent membrane was scanned at a wavelength range of 300nm to 700nm using the POWERSCAN HT (DS Pharma Biomedical Co., Ltd). The absorbance in a range of wavelength measurements of the transparent membranes obtained in Examples 4 to 8 were shown in Figure 8. In the cross-linked transparent membranes, the absorbance in a range of wavelengths of 300nm to 380nm was reduced in a linear manner. On the other hand, the absorbance in a range of wavelengths of 500nm to 800nm was unchanged.

<<EXAMPLE 9: Preparation of non-fibrogenesis collagen porous material>>

In this example, the collagen porous material was prepared using a collagen acid solution containing carbon dioxide.

The acid solution at pH 4 or less was prepared by bubbling carbon dioxide in distilled water. The lyophilized collagen obtained in Preparing Example 1 was dissolved in the acid solution so that the concentration became 5% by weight. The resulting collagen solution was poured into a cell culture plate (48 well) so that a height of the collagen solution became 2cm, and was stored in a refrigerator at 4°C. Subsequently, the collagen solution was frozen by gradually cooling to -20°C. After complete freezing, this frozen collagen solution was dried by a freeze dryer. The appearance of the resulting porous, collagen material is shown in Figure 9(a).

Then, the obtained collagen porous material was thermally cross-linking. Specifically, said material was thermally cross-linked by being allowed to stand in a vacuum dryer at 130°C for 24 hours. The appearance of the resulting, thermally cross-linked, collagen porous material is shown in Figure 9(b).

Further, the thermally cross-linked collagen porous material was chemically cross-linked. Said resulting material and a 25% glutaraldehyde (GA) solution were placed in a desiccator, and the material was treated at 37 °C for 4 hours, in a vacuum. The appearance of the resulting GA cross-linked collagen porous material is shown in Figure 9(c). A porosity of the resulting porous material is 95%.

The average pore diameter of the resulting porous material was analyzed by measuring diameters of 100 pores at 1000 fold magnification according to an observation with a scanning electron microscope. The average pore diameter of the resulting porous material was 150$\mu$m.

(Water permiability test)

The water permiability test was carried out by adding a drop of distilled water to the surface of the obtained collagen porous material. The water permiability is important as an index of cell penetration into the collagen porous material in culturing cells. The GA cross-linked collagen porous material (c) absorbed the drop of distilled water just after dropping (Figure 10A). As shown in Figure 10A, the water permiability of said material without cross-linking (a) and the thermally cross-linked collagen porous material (b) were poor.

The photographs of the above collagen porous materials observed after 10 minutes are shown in Figure 10B. As shown in Figure 10B, the GA cross-linked collagen porous material (c) and the thermally cross-linked, porous, collagen material (b) completely absorbed the drop of distilled water. Particularly, in the GA cross-linked collagen porous material (c), water was penetrated to the lower surface thereof.

<<EXAMPLE 10>>

The acid solution (pH 4 or less) was prepared by bubbling carbon dioxide in distilled water. The lyophilized collagen obtained in Preparing Example 1 was dissolved in the acid solution so that the concentration became 1% by weight. The resulting collagen solution added dropwise to a cell culture dish with a diameter of 33mm so that a height of the collagen solution became 2mm. The collagen solution in the cell culture dish was allowed to stand over night at 4°C, so as to smooth the surface thereof. Further, the collagen solution was frozen by gradually cooling to -20°C. After complete freezing, the frozen collagen solution was dried by a freeze dryer, to thereby obtain a collagen porous material.

Then, the said resulting material was thermally cross-linked. Specifically, the collagen porous material was thermally cross-linked by being allowed to stand in a vacuum dryer at 130°C for 24 hours, to obtain a thermally cross-linked collagen porous material.

Further, the thermally cross-linked collagen porous material was chemically cross-linked. The obtained material and 100% glutaraldehyde (GA) were placed in a desiccator, and the material was treated at 37 °C for 24 hours, in a vacuum. A porosity of the resulting collagen porous material is 99%.

The average pore diameter of the resulting collagen porous material was analyzed by measuring the diameters of 100 pores at 1000 fold magnification according to an observation by a scanning electron microscope. The average pore diameter of the resulting collagen porous material was 100$\mu$m.

(Measurement of viscoelastic property)

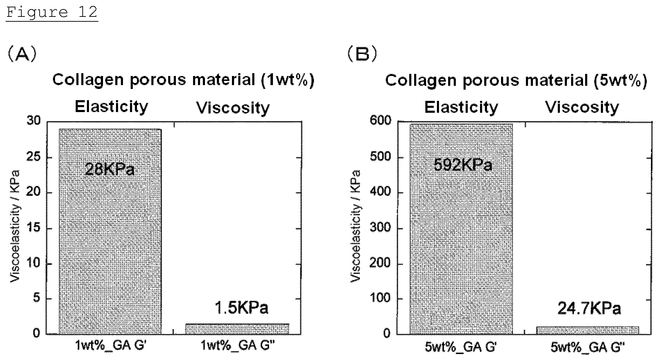

A viscoelastic property was measured in the condition wherein the manufactured GA cross-linked collagen porous materials were immersed in distilled water. The measurement was carried out at a range with linear viscoelasticity. A measured frequency range was 0.01Hz to 10Hz. The GA cross-linked collagen porous material prepared using 1% by weight of a collagen solution exhibits 28kPa of elasticity and 1.5kPa of viscosity at a 1Mz of frequency (Figure 12A).

(Scanning electron micrograph)

A scanning electron micrograph of the manufactured GA cross-linked collagen porous material is shown in Figure 11A. The region, wherein a structure was observed, was a cut, central portion of the collagen porous material. The observed pore diameters thereof were 100$\mu$m or more. Therefore, it is found that said material with such pore diameters is most appropriate as the cell culture substrate or the implantation material.

«EXAMPLE 11»

The procedures of Example 10 were repeated, except that 5% by weight of the collagen solution was used instead of 1% by weight of the collagen solution, to obtain the collagen porous material, thermally cross-linked, collagen porous material, and the GA cross-linked collagen porous material.

(Measurement of viscoelastic property)

A viscoelastic property was measured in the condition wherein the manufactured GA cross-linked collagen porous materials were immersed in distilled water. The measurement was carried out at a range inlinear viscoelasticity. A measured frequency range was 0.01Hz to 10Hz. The GA cross-linked collagen porous material prepared using 5% by weight of collagen solution exhibits 592kPa of elasticity value and 24.7kPa of viscosity value at a 1Mz of frequency (Figure 12B).

(Scanning electron micrograph)

A scanning electron micrograph of the GA cross-linked collagen porous material is shown in Figure 11B. The region, wherein a structure was observed, was a cut, central portion of the collagen porous material. The observed pore diameters thereof were $100\mu m$ or more. Therefore, it is found that the collagen porous material with such pore diameters is most appropriate as the cell culture substrate or the implantation material. Further, it is found that the porous structure is maintained, in spite of the change from 1% by weight of a collagen solution to 5% by weight of a collagen solution. Furthermore, when the collagen concentrations become higher, the pore diameters become smaller.

INDUSTRIAL APPLICABILITY

The non-fibrogenesis collagen material of the present invention can be used as a cell culture substrate, a scaffold material for regenerative medicine (material for tissue engineering of cartilage, bone, ligament, corneal stroma, skin, or liver, for example), an implantation material (wound dressing material, bone grafting material, hemostatic material, or anti-adhesive material, for example) or a carrier for drug delivery.

Although the present invention has been described with reference to specific embodiments, various changes and modifications obvious to those skilled in the art are possible without departing from the scope of the appended claims.

**Claims**

1.  A non-fibrogenesis collagen transparent membrane **characterized by** comprising a fish-derived collagen, wherein collagen molecules form nano fibers, and the nano fibers do not form collagen fine fibrils, the density determined by the gravimetric method is 0.4 g/cm$^3$ or more, and the tensile strength is 30 MPa or more.

2.  The non-fibrogenesis collagen transparent membrane according to claim 1, wherein the fish-derived collagen is derived from a fish scale.

3.  The non-fibrogenesis collagen transparent membrane according to claim 1 or 2, wherein a surface roughness of the non-fibrogenesis collagen transparent membrane is 30 nm or less.

4.  The non-fibrogenesis collagen transparent membrane according to claim any one of claims 1 to 3, wherein the non-fibrogenesis collagen transparent membrane transmits 90% or more of lights with wavelengths ranging from 500 nm to 700 nm.

5.  The non-fibrogenesis collagen transparent membrane according to any one of claims 1 to 4, wherein the collagen is cross-linked, and the tensile strength is 50 MPa or more.

6.  A method for preparing a non-fibrogenesis collagen transparent membrane, comprising the steps of:

    (1) preparing a collagen acid solution containing carbon dioxide and fish-derived collagen, and
    (2) forming a collagen transparent membrane from the collagen acid solution.

7.  The method for preparing a non-fibrogenesis collagen transparent membrane according to claim 6, comprising the steps of:

    (1) preparing a collagen acid solution containing carbon dioxide and fish-derived collagen,
    (2) forming a collagen transparent membrane from the collagen acid solution, and
    (3) drying the collagen transparent membrane.

8. The method for preparing a non-fibrogenesis collagen transparent membrane according to claim 6 or 7, comprising the steps of:

   (1) preparing a collagen acid solution containing carbon dioxide and fish-derived collagen,
   (2) forming a collagen transparent membrane from the collagen acid solution,
   (3) drying the collagen transparent membrane, and
   (4) cross-linking the dried collagen transparent membrane.

9. The method for preparing a non-fibrogenesis collagen transparent membrane according to any one of claims 6 to 8, the fish-derived collagen is derived from a fish scale.

10. The method for preparing a non-fibrogenesis collagen transparent membrane according to any one of claims 6 to 9, wherein the collagen transparent membrane is a non-fibrogenesis collagen porous transparent membrane.

**Patentansprüche**

1. Transparente Membran aus Nicht-Fibrogenese-Kollagen, **dadurch gekennzeichnet, dass** diese aus Fisch gewonnenes Kollagen umfasst, wobei die Kollagenmoleküle Nanofasern bilden und die Nanofasern keine feinen Kollagenfibrillen bilden, wobei die Dichte, die mittels gravimetrischer Analyse bestimmt wird, 0,4 g/cm$^3$ oder mehr ist und die Zugfestigkeit 30 MPa oder mehr ist.

2. Die transparente Membran aus Nicht-Fibrogenese-Kollagen nach Anspruch 1, wobei das aus Fisch gewonnene Kollagen aus einer Fischschuppe gewonnen wird.

3. Die transparente Membran aus Nicht-Fibrogenese-Kollagen nach Anspruch 1 oder 2, wobei die Oberflächenrauhigkeit der transparenten Membran aus Nicht-Fibrogenese-Kollagen30 nm oder weniger ist.

4. Die transparente Membran aus Nicht-Fibrogenese-Kollagen nach einem der Ansprüche 1 bis 3, wobei die transparente Membran aus Nicht-Fibrogenese-Kollagen90% oder mehr von Licht mit einer Wellenlänge von 500 nm bis 700 nm durchlässt.

5. Die transparente Membran aus Nicht-Fibrogenese-Kollagen nach einem der Ansprüche 1 bis 4, wobei das Kollagen vernetzt ist und die Zugfestigkeit 50 MPa oder mehr ist.

6. Verfahren zur Herstellung einer transparenten Membran aus Nicht-Fibrogenese-Kollagen, welches die Schritte

   (1) Herstellen einer sauren Kollagenlösung, die Kohlendioxid und aus Fisch gewonnenes Kollagen enthält, und
   (2) Bilden einer transparenten Kollagenmembran aus der sauren Kollagenlösung

   umfasst.

7. Das Verfahren zur Herstellung einer transparenten Membran aus Nicht-Fibrogenese-Kollagen nach Anspruch 6, welches die Schritte

   (1) Herstellen einer sauren Kollagenlösung, die Kohlendioxid und aus Fisch gewonnenes Kollagen enthält,
   (2) Bilden einer transparenten Kollagenmembran aus der sauren Kollagenlösung, und
   (3) Trocknen der transparenten Kollagenmembran

   umfasst.

8. Das Verfahren zur Herstellung einer transparenten Membran aus Nicht-Fibrogenese-Kollagen nach Anspruch 6 oder 7, welches die Schritte

   (1) Herstellen einer sauren Kollagenlösung, die Kohlendioxid und aus Fisch gewonnenes Kollagen enthält,
   (2) Bilden einer transparenten Kollagenmembran aus der sauren Kollagenlösung,
   (3) Trocknen der transparenten Kollagenmembran, und
   (4) Vernetzen der getrockneten transparenten Kollagenmembran

umfasst.

9. Das Verfahren zur Herstellung einer transparenten Membran aus Nicht-Fibrogenese-Kollagen nach einem der Ansprüche 6 bis 8, wobei das aus Fisch gewonnene Kollagen aus einer Fischschuppe gewonnen wird.

10. Das Verfahren zur Herstellung einer transparenten Membran aus Nicht-Fibrogenese-Kollagen nach einem der Ansprüche 6 bis 9, wobei die transparente Kollagenmembran eine poröse transparente Membran aus Nicht-Fibrogenese-Kollagen ist.

**Revendications**

1. Membrane transparente de collagène non fibrogène **caractérisée en ce qu'**elle comprend un collagène dérivé du poisson, dans laquelle les molécules de collagène forment des nanofibres, et les nanofibres ne forment pas de fibrilles fines de collagène, la densité déterminée par la méthode gravimétrique est supérieure ou égale à 0,4 g/cm$^3$, et la résistance à la traction est supérieure ou égale à 30 MPa.

2. Membrane transparente de collagène non fibrogène selon la revendication 1, dans laquelle le collagène dérivé du poisson est dérivé d'une écaille de poisson.

3. Membrane transparente de collagène non fibrogène selon la revendication 1 ou 2, dans laquelle une rugosité de surface de la membrane transparente de collagène non fibrogène est inférieure ou égale à 30 nm.

4. Membrane transparente de collagène non fibrogène selon l'une quelconque des revendications 1 à 3, dans laquelle la membrane transparente de collagène non fibrogène transmet 90% ou plus de lumières ayant des longueurs d'onde se trouvant dans une plage allant de 500 nm à 700 nm.

5. Membrane transparente de collagène non fibrogène selon l'une quelconque des revendications 1 à 4, dans laquelle le collagène est réticulé, et la résistance à la traction est supérieure ou égale à 50 MPa.

6. Procédé pour préparer une membrane transparente de collagène non fibrogène, comprenant les étapes qui consistent :

   (1) à préparer une solution acide de collagène contenant du dioxyde de carbone et du collagène dérivé du poisson, et
   (2) à former une membrane transparente de collagène à partir de la solution acide de collagène.

7. Procédé pour préparer une membrane transparente de collagène non fibrogène selon la revendication 6, comprenant les étapes qui consistent :

   (1) à préparer une solution acide de collagène contenant du dioxyde de carbone et du collagène dérivé du poisson,
   (2) à former une membrane transparente de collagène à partir de la solution acide de collagène, et
   (3) à sécher la membrane transparente de collagène.

8. Procédé pour préparer une membrane transparente de collagène non fibrogène selon la revendication 6 ou 7, comprenant les étapes qui consistent :

   (1) à préparer une solution acide de collagène contenant du dioxyde de carbone et du collagène dérivé du poisson,
   (2) à former une membrane transparente de collagène à partir de la solution acide de collagène,
   (3) à sécher la membrane transparente de collagène, et
   (4) à réticuler la membrane transparente de collagène séchée.

9. Procédé pour préparer une membrane transparente de collagène non fibrogène selon l'une quelconque des revendications 6 à 8, le collagène dérivé du poisson est dérivé d'une écaille de poisson.

10. Procédé pour préparer une membrane transparente de collagène non fibrogène selon l'une quelconque des revendications 6 à 9, dans lequel la membrane transparente de collagène est une membrane transparente poreuse de collagène non fibrogène.

Figure 1

Figure 2

Figure 3

Figure 4

(A)

10kV X5,000 5μm 000080

(B)

10kV X5,000 5μm 000088

Figure 5

（A）

36.45
[nm]

0.00

2.00 µm          5.00 x 5.00 µm

-5.55
[V]

-5.86

2.00 µm          5.00 x 5.00 µm

（B）

23.73
[nm]

0.00

1.00 µm          2.50 x 2.50 µm

-7.26
[V]

-7.83

1.00 µm          2.50 x 2.50 µm

Figure 6

（A）

43.45
[nm]

0.00

2.00 µm          5.00 x 5.00 µm
透明膜-02

（B）

48.26
[nm]

0.00

2.00 µm          5.00 x 5.00 µm
ブタ透明膜pH3.8

Figure 7

(A)

(B)

(C)

Figure 8

Figure 9

(a)　　　　　　(b)　　　　　　(c)

Figure 10

(A)

(a)　　　　　　(b)　　　　　　(c)

(B)

(a)　　　　　　(b)　　　　　　(c)

Figure 11

(A)　　　　　　　　　　　　　　(B)

Figure 12

(A)

**Collagen porous material (1wt%)**

(B)

**Collagen porous material (5wt%)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003534858 A
- JP 2005334625 A
- WO 0192322 A1
- JP 2006257014 A

**Non-patent literature cited in the description**

- *YAKUGAKU ZASSHI,* 2010, vol. 130, 565-574
- *Journal of BIOTECHNOLOGY,* 2007, vol. 131, 76-83
- **CHEN.** *Acta Biomaterialia,* 2011, vol. 7, 644-652
- *Biochemical and Biophysical Research Communications,* 1972, vol. 2, 320-325